(19) Europäisches Patentamt
European Patent Office
Office européen des brevets

(11) **EP 1 606 241 B1**

(12) **EUROPÄISCHE PATENTSCHRIFT**

(45) Veröffentlichungstag und Bekanntmachung des Hinweises auf die Patenterteilung:
**19.03.2008 Patentblatt 2008/12**

(21) Anmeldenummer: **04717071.7**

(22) Anmeldetag: **04.03.2004**

(51) Int Cl.:
***C07C 47/058*** (2006.01)

(86) Internationale Anmeldenummer:
**PCT/EP2004/002180**

(87) Internationale Veröffentlichungsnummer:
**WO 2004/078678 (16.09.2004 Gazette 2004/38)**

(54) **VERFAHREN ZUR ERZEUGUNG HOCHKONZENTRIERTER FORMALDEHYDL SUNG EN**

METHOD FOR THE PRODUCTION OF HIGHLY CONCENTRATED FORMALDEHYDE SOLUTIONS

PROCEDE DE PRODUCTION DE SOLUTIONS DE FORMALDEHYDE HAUTEMENT CONCENTREES

(84) Benannte Vertragsstaaten:
**AT BE BG CH CY CZ DE DK EE ES FI FR GB GR HU IE IT LI LU MC NL PL PT RO SE SI SK TR**

(30) Priorität: **04.03.2003 DE 10309289**

(43) Veröffentlichungstag der Anmeldung:
**21.12.2005 Patentblatt 2005/51**

(73) Patentinhaber: **BASF SE**
**67056 Ludwigshafen (DE)**

(72) Erfinder:
• **STROEFER, Eckhard**
**68163 Mannheim (DE)**
• **LANG, Neven**
**68163 Mannheim (DE)**
• **HASSE, Hans**
**67661 Kaiserslautern (DE)**
• **GRUETZNER, Thomas**
**70567 Stuttgart (DE)**
• **OTT, Michael**
**69151 Neckargemünd (DE)**

(74) Vertreter: **Hörschler, Wolfram Johannes et al**
**Isenbruck, Bösl, Hörschler, Wichmann, Huhn**
**Theodor-Heuss-Anlage 12**
**68165 Mannheim (DE)**

(56) Entgegenhaltungen:
**DE-A- 10 062 814     GB-A- 1 190 682**

**Beschreibung**

Die Erfindung betrifft ein Verfahren zur Herstellung hochkonzentrierter Formaldehydlösungen.

Formaldehyd ist eine wichtige Industriechemikalie und wird zur Herstellung zahlreicher Industrieprodukte und Verbrauchsartikel eingesetzt. In über 50 Industriezweigen wird derzeit Formaldehyd verwendet, im Wesentlichen in Form von wässrigen Lösungen oder Formaldehyd enthaltenden Kunstharzen. Kommerziell erhältliche, wässrige Formaldehyd Lösungen weisen Gesamtkonzentrationen von 20 bis 55 Gew.-% Formaldehyd in Form von monomerem Formaldehyd, Methylenglykol und oligomeren Polyoxymethylenglykolen auf.

Wasser, monomerer (freier) Formaldehyd, Methylenglykol und oligomere Polyoxymethylenglykole unterschiedlicher Kettenlänge liegen in wässrigen Lösungen nebeneinander in einem thermodynamischen Gleichgewicht vor, das durch eine bestimmte Verteilung der Polyoxymethylenglykole unterschiedlicher Länge gekennzeichnet ist. Der Begriff "wässrige Formaldehydlösung" bezieht sich dabei auch auf Formaldehydlösungen, die praktisch kein freies Wasser, sondern im Wesentlichen nur noch in Form von Methylenglykol bzw. in den enständigen OH-Gruppen der Polyoxymethylenglykole chemisch gebundenes Wasser enthalten. Dies ist insbesondere bei konzentrierten Formaldehydlösungen der Fall. Polyoxymethylenglykole können dabei beispielsweise zwei bis neun Oxymethyleneinheiten aufweisen. In wässrigen Formaldehydlösungen können also Dioxymethylenglykol, Trioxymethylenglykol, Tetraoxymethylenglykol, Pentaoxymethylenglykol, Hexaoxymethylenglykol, Heptaoxymethylenglykol, Octaoxymethylenglykol und Nonaoxymethylenglykol nebeneinander vorliegen. Die Verteilung ist konzentrationsabhängig. So liegt das Maximum der Verteilung in verdünnten Formaldehydlösungen bei Homologen niedriger Kettenlänge, während es in konzentrierteren Formaldehydlösungen bei Homologen höherer Kettenlänge liegt. Eine Gleichgewichtsverschiebung hin zu längerkettigen (höhermolekularen) Polyoxymethylenglykolen kann durch Wasserentzug, beispielsweise durch einfache Destillation in einem Filmverdampfer, erfolgen. Die Gleichgewichtseinstellung erfolgt dabei mit endlicher Geschwindigkeit durch die intermolekulare Kondensation von Methylenglykol und niedermolekularen Polyoxymethylenglykolen unter Wasserabspaltung zu höhermolekularen Polyoxymethylenglykolen.

Die durch Wasserentzug erhaltenen hochkonzentrierten Formaldehydlösungen sind jedoch in dem Sinne instabil, dass nach einer bestimmten Zeit ein Feststoffausfall auftritt. Die ausfallenden Feststoffe sind im Wesentlichen die oben beschriebenen längerkettigen Formaldehyd-Oligomere oder Polyoxymethylenglykole. Es ist bekannt, mäßig konzentrierte Formaldehydlösungen mit $CH_2O$-Gehalten bis ca. 50 Gew.-% mit ca. 0,2 bis 2 Gew.-% Methanol als Stabilisator zu versetzen und bei ca. 55 °C zu lagern, um einen Feststoffausfall zu vermeiden. Höher konzentrierte Formaldehydlösungen mit > 70 Gew.-% $CH_2O$, beispielsweise ca. 80 Gew.-% $CH_2O$, fallen bei der Herstellung bei niedrigen Temperaturen von ca. 20 bis 50 °C zunächst einphasig an. Nach einer gewissen Zeit kommt es jedoch zum Feststoffausfall. Ursache scheint das Anwachsen der Polyoxymethylenglykol-Ketten in der Formaldehydlösung bis zur Überschreitung der Löslichkeitsgrenze zu sein. GB 1,190,682 beschreibt ein Verfahren zur Herstellung Konzentrierter, wässriger Formaldehydlösungen.

Aufgabe der Erfindung ist es, ein Verfahren zur Herstellung hochkonzentrierter wässriger Formaldehydlösungen mit einem $CH_2O$-Gehalt von ≥ 50 Gew.-% bereitzustellen, bei dem ein Feststoffausfall entweder ganz vermieden wird oder nur in untergeordnetem Maße auftritt. Der Feststoffausfall soll in jedem Fall so gering sein, dass die resultierende Suspension in technischen Apparaten gefördert werden kann. Im Allgemeinen ist dies bei einem Feststoffgehalt von bis zu 10 Gew.-% der Fall.

Gelöst wird die Aufgabe durch ein Verfahren zur Herstellung hochkonzentrierter Formaldehydlösungen mit einem $CH_2O$-Gehalt ≥ 50 Gew.-% aus einer wässrigen Formaldehydlösung mit niedrigerem $CH_2O$-Gehalt durch Verdampfen eines Teils dieser Lösung (partielle Verdampfung), bei dem die wässrige Formaldehydlösung auf eine Verdampfungstemperatur T erwärmt wird, bei der sich Wasser in der Gasphase relativ zur flüssigen Phase anreichert, und die gebildete Gasphase kontinuierlich oder diskontinuierlich abgezogen wird, wobei für die Verdampfungstemperatur T gilt:

$$T \ [°C] \ < T_{max} \ [°C]$$

mit

$$T_{max}(c) = A + B \times (c/100) + C \times (c/100)^2 + D \times (c/100)^3$$

und

$$A = + 68,759, B = + 124,77, C = - 12,851, D = - 10,095.$$

wobei c der aktuelle $CH_2O$-Gehalt der wässrigen Formaldehydlösung während der Verdampfung in Gew.-% ist und von 20 bis 99 Gew.-% beträgt.

Bevorzugt wird von einer wässrigen Formaldehydlösung mit einem $CH_2O$-Gehalt von 10 bis 95 Gew.-%, besonders bevorzugt von 30 bis 85 Gew.-% ausgegangen. Diese können durch oxidative Methanol-Dehydrierung und gegebenenfalls nachfolgender Aufkonzentrierung der erhaltenen wässrigen Formaldehydlösung erhalten werden. Mit dem erfindungsgemäßen Verfahren lassen sich hochkonzentrierte Formaldehydlösungen mit einem $CH_2O$-Gehalt von > 80 Gew.-% oder sogar von > 95 Gew.-% bis zu 99 Gew.-% erhalten.

Die Verdampfung kann in handelsüblichen Apparaten erfolgen. Geeignet sind beispielsweise Rührkessel, die z.B. durch Doppelmäntel oder Rohrschlangen (innenliegend oder außen angebracht) geheizt werden können. Besonders geeignet sind Apparate mit Wärmetauschercharakteristik, z.B. Rohrbündelwärmetauscher, Plattenapparate oder Wendelrohre. Diese können im Gleichstrom, Gegenstrom- oder Kreuzstrom betrieben werden. Die Beheizung kann durch beliebige Medien erfolgen, beispielsweise mit kondensierendem Dampf oder einphasig durch Flüssigkeiten oder Gase. Die Verdampfung der wässrigen Formaldehydlösung kann in einmaligem Durchgang durch den Verdampfer oder im Umlauf erfolgen. Insbesondere dann, wenn vollständige Verdampfung angestrebt wird, wird ein einmaliger Durchlauf der Formaldehydlösung durch den Verdampfer im Allgemeinen nicht ausreichen.

Die Verdampfung kann auch in einer Kolonne, beispielsweise einer Glockenbodenkolonne, durchgeführt werden.

Die Formaldehydlösung wird vorzugsweise bei einer Temperatur verdampft, bei der kein Feststoff ausfällt. Besonders bevorzugt ist es, an jeder Stelle in dem Verdampfers eine Temperatur aufrecht zu erhalten, bei der kein Feststoff ausfällt. Beispielsweise wird diese Temperatur sowohl in dem Verdampfer selbst als auch - bei Betrieb des Verdampfers im Umlauf - in dem Umlauf und bei Entnahme der wässrigen Formaldehydlösung in den dem Verdampfer nachgeschalteten Einrichtungen aufrechterhalten.

Dazu wird an jeder Stelle in dem Verdampfer eine Temperatur

$$T \, [°C] > T_{min} \, [°C]$$

mit

$$T_{min}(c) = A' + B' \times (c/100) + C' \times (c/100)^2 + D' \times (c/100)^3$$

und
mit

$$A' = + 6,0156, B' = + 52,918, C' = + 49,699, D' = + 34,286,$$

wobei c der aktuelle $CH_2O$-Gehalt der wässrigen Formaldehydlösung während der Verdampfung in Gew.-% ist und 20 bis 99 Gew.-% beträgt, in der wässrigen Formaldehydlösung aufrechterhalten.

Es wurde überraschender Weise gefunden, dass sich wässrige Formaldehydlösungen durch partielles Verdampfen aufkonzentrieren lassen, ohne dass Feststoffausfall eintritt, wenn in dem oben definierten Temperaturfenster gearbeitet wird.

Die Abreicherung von Wasser in der flüssigen Phase kann damit erklärt werden, dass durch Kondensation von Polyoxymethylenglykolen untereinander oder von Polyoxymethylenglykolen mit Methylenglykol Wasser freigesetzt wird.

Die Verdampfung der wässrigen Formaldehydlösung kann in Gegenwart saurer oder basischer Katalysatoren durchgeführt werden, welche die oben skizzierten Kondensationsreaktionen katalysieren. Jedoch wird man aus Kostengründen, und auch um Ablagerungen an den Wärmetauscherflächen zu vermeiden, die Katalysatorzusätze gering halten. Die Katalyse kann homogen oder auch heterogen in Suspensions- oder Festbettfahrweise erfolgen.

Im Allgemeinen liegt der Druck während der partiellen Verdampfung im Bereich von 0,02 bis 50 bar, bevorzugt im Bereich von 0,1 bis 17 bar.

Die partielle Verdampfung kann kontinuierlich oder diskontinuierlich erfolgen. Diskontinuierlich erfolgt die partielle Verdampfung beispielsweise in einem einfachen Rührkessel. Die partielle Verdampfung kann auch kontinuierlich in einem Filmverdampfer oder Dünnschichtverdampfer erfolgen, wie er in EP-A 1 063 221 beschrieben ist, oder auch in einem Wendelrohrverdampfer, wie er in DE-A 27 19 967 beschrieben ist.

Das erfindungsgemäße Verfahren geht vorzugsweise von mäßig oder hochkonzentrierten Formaldehydlösungen mit einem $CH_2O$-Gehalt von 30 bis 95 Gew.-% aus, die nach ihrer Herstellung wie nachfolgend beschrieben gegen Feststoffausfall stabilisiert wurden.

Höher konzentrierte Formaldehydlösungen mit beispielsweise > 70 Gew.-% $CH_2O$ fallen bei der Herstellung bei niedrigen Temperaturen von ca. 20 bis 50 °C zunächst einphasig an. Nach einer gewissen Zeit kommt es jedoch zum Feststoffausfall. Ursache scheint das Anwachsen der Polyoxymethylenglykol-Ketten in der Formaldehydlösung bis zur Überschreitung der Löslichkeitsgrenze zu sein. Diese Lösungen können gegen Feststoffausfall stabilisiert werden, indem sie unmittelbar nach der Herstellung mit einer Aufheizrate von mindestens 5°C/min auf eine Temperatur von mindestens 80 °C bis höchstens 200 °C erwärmt und bei einer Temperatur in diesem Bereich belassen werden. Unmittelbar nach der Herstellung bedeutet, dass die beispielsweise bei 20 bis 60°C erhaltene hochkonzentrierte Formaldehydlösung nach spätestens 60 min, bevorzugt nach spätestens 5 min, mit der spezifizierten Aufheizrate erwärmt wird.

Vorzugsweise beträgt die Aufheizrate mindestens 10 °C/min. Eine Aufheizrate von mindestens 10°C/min ist insbesondere dann bevorzugt, wenn der pH-Wert der Lösung < 3 oder > 6 beträgt. Vorzugsweise wird mit der spezifizierten Aufheizrate auf mindestens 100 °C erwärmt und diese Temperatur anschließend nicht mehr unterschritten. Der pH-Wert der hochkonzentrierten Formaldehydlösung liegt üblicherweise im Bereich von 1 bis 10, vorzugsweise 2 bis 9, besonders bevorzugt von 3 bis 6. Der pH-Wert kann durch Zugabe von Puffersubstanzen, beispielsweise einem Formiat-Puffer, auf den gewünschten Bereich eingestellt werden.

Vorzugsweise werden Stabilisierung und Verdampfung der hochkonzentrierten wässrigen Formaldehydlösungen in einem Apparat durchgeführt.

Die erhaltenen hochkonzentrierten Formaldehydlösungen können für eine Vielzahl chemischer Umsetzungen verwendet werden. Beispiele für derartige Umsetzungen sind

- die Umsetzung von Acetylen mit Formaldehydlösung in einer Reppe-Reaktion zu Butindiol, das zu Butandiol weiter hydriert werden kann;

- Aldolisierungsreaktionen von Formaldehyd mit sich selbst oder höheren Aldehyden zu mehrwertigen Alkoholen und Zuckern, Pentaerythrit, Trimethylolpropan und Neopentylglykol;

- die Umsetzung von Formaldehyd und CO zu Glykolsäure;

- die Herstellung chelatisierender Substanzen wie Glykolnitrile aus Lösungen von Formaldehyd;

- die Umsetzung von Formaldehyd mit Olefinen in einer Prins-Reaktion zu alpha-Hydroxymethylverbindungen;

- Kondensationsreaktionen von Formaldehyd mit Aminen, wie Anilin oder Toluidin, zu Schiffschen Basen, die zu Diphenylmethanderivaten wie Methandiphenyldiamin weiterreagieren können;

- Umsetzung von Hydroxylamin mit Formaldehyd zu Oximen;

- Umsetzung von Formaldehyd mit Diolen zu cyclischen Ethern, beispielsweise von Glykol und Formaldehyd zu Dioxolan.

Die Listung ist nicht vollständig. Lehrbücher der organischen Chemie und der technischen Chemie enthalten weitere Beispielreaktionen. Die Listung soll aber beispielhaft die industrielle Bedeutung des Formaldehyds als Synthesebaustein im gesamten Bereich der organischen Chemie verdeutlichen. Dies betrifft sowohl kleintonnagige Zwischenprodukte im Pharma- oder Pflanzenschutzbereich wie z.B. Oxime als auch großtonnagige Produkte wie Diphenylmethanderivate.

Besonders bevorzugt wird die erfindungsgemäß hergestellte hochkonzentrierte Formaldehydlösung zur Herstellung von Polyoxymethylen-Kunststoffen verwendet. Die Herstellung von Polyoxymethylen-Kunststoffen ist beispielsweise in der nicht vorveröffentlichten deutschen Patentanmeldung DE 101 58 813.5 beschrieben. Weiterhin ist die Verwendung der hochkonzentrierten Formaldehydlösung für die Herstellung von Trioxan oder Tetraoxan in flüssiger Phase bevorzugt. Weiterhin bevorzugt ist die Verwendung der hochkonzentrierten Formaldehydlösung zur Herstellung von Polyoxymethylendialkylethem durch Kondensation mit Alkoholen.

Die Erfindung wird durch die nachstehenden Beispiele näher erläutert.

**Beispiele**

**Beispiel 1**

In einem Laborversuch wird in einem Dünnschichtverdampfer im geraden Durchgang eine hochkonzentrierte Formaldehydlösung hergestellt. Der Verdampfer weist eine Verdampferfläche von 0,092 m$^2$ und eine Länge von 1,1 m auf. Am Kopf wird eine 48 gew.-%ige wässrige Formalinlösung aufgegeben. Der Mengenstrom beträgt 615 g/h. Die Wandtemperatur beträgt 90 °C, der Druck beträgt 80 mbar. Am Sumpf werden 298 g/h einer 84 gew.-%igen hochkonzentrierten Formaldehydlösung abgezogen. Am Kopf werden 321 g/h Brüden abgezogen. Die Sumpflösung wird mit einer Laborpumpe in einen geheizten Standard-Laborrührreaktor mit 1 1 Inhalt gefördert. Der Reaktor wird auf einer Innentemperatur von 130 °C gehalten. Aus dem Reaktor wird ein Flüssigkeitsstrom von 64 g/h entnommen. Aus der Gasphase des Reaktors wird ein Gasstrom von 234 g/h abgezogen. Der Flüssigkeitsstrom enthält nach Analyse 85,1 Gew.-% Formaldehyd, berechnet als $CH_2O$.

**Beispiel 2**

In einem Laborversuch wird in einer Destillationskolonne eine hochkonzentrierte Formaldehydlösung aufgearbeitet. Bei der Kolonne handelt es sich um eine Glockenbodenkolonne mit 20 Stufen und einen Innendurchmesser von 50 mm. Der Kopfdruck der Kolonne beträgt 2,0 bar.

Der Kolonne wird auf der 10. Stufe eine Formaldehydlösung mit 84 Gew.-% Formaldehyd zugeführt. Der Mengenstrom beträgt 2,8 kg/h, die Temperatur 107°C. Am Kopf der Kolonne werden 0,93 kg/h abgezogen. Der Formaldehyd-Gehalt des Kopfabzugsstroms beträgt 77,2Gew.-%. Am Sumpf der Kolonne werden 1,85 kg/h einer aufkonzentrierten Lösung mit einem Formaldehyd-Gehalt von 87,3 Gew.-% abgezogen. Die Temperatur des Sumpfabzugstroms beträgt 122°C.

**Patentansprüche**

1. Verfahren zur Herstellung hochkonzentrierter Formaldehydlösungen mit einem $CH_2O$-Gehalt $\geq$ 50 Gew.-% aus einer wässrigen Formaldehydlösung mit niedrigerem $CH_2O$-Gehalt durch Verdampfen eines Teils dieser Lösung (partielle Verdampfung), bei dem die wässrige Formaldehydlösung auf eine Verdampfungstemperatur T erwärmt wird, bei der sich Wasser in der Gasphase relativ zur flüssigen Phase anreichert, und die gebildete Gasphase kontinuierlich oder diskontinuierlich abgezogen wird, wobei für die Verdampfungstemperatur T gilt:

$$T \,[°C] < T_{max} \,[°C]$$

mit

$$T_{max}(c) = A + B \times (c/100) + C \times (c/100)^2 + D \times (c/100)^3$$

und A = +68,759, B = +124,77, C = -12,851, D = -10,095,
wobei
an jeder Stelle in dem Verdampfer eine Temperatur

$$T \,[°C] > T_{min} \,[°C]$$

mit

$$T_{min}(c) = A' + B' \; x \; (c/100) + C' \; x \; (c/100)^2 + D' \; x \; (c/100)^3$$

und
mit A'=+6,0156, B'=+52,918, C'=+49,699, D'=+34,286,
in der wässrigen Formaldehydlösung aufrechterhalten wird, wobei c der aktuelle $CH_2O$-Gehalt der wässrigen Formaldehydlösung während der Verdampfung in Gew.-% ist und von 20 bis 99 Gew.-% beträgt.

**2.** Verfahren nach Anspruch 1, **dadurch gekennzeichnet, dass** die wässrige Formaldehydlösung, von der das Verfahren ausgeht, einen $CH_2O$-Gehalt von 10 bis 95 Gew.-% aufweist.

**3.** Verfahren nach Anspruch 2, **dadurch gekennzeichnet, dass** die wässrige Formaldehydlösung, von der das Verfahren ausgeht, einen $CH_2O$-Gehalt von 30 bis 85 Gew.-% aufweist.

**4.** Verfahren nach einem der Ansprüche 1 bis 3, **dadurch gekennzeichnet, dass** der Druck während der partiellen Verdampfung von 0,1 bis 50 bar beträgt.

**5.** Verfahren nach einem der Ansprüche 1 bis 4, **dadurch gekennzeichnet, dass** die erhaltene hochkonzentrierte Formaldehydlösung einen $CH_2O$-Gehalt von 50 bis 99 Gew.-% aufweist.

**6.** Verfahren nach einem der Ansprüche 1 bis 5, **dadurch gekennzeichnet, dass** die Verdampfung der wässrigen Formaldehydlösung in einem Rührkessel, einem Wendelrohr, einem Filmverdampfer oder einem anderen Apparat mit Wärmetauschercharakteristik durchgeführt wird.

**7.** Verfahren nach einem der Ansprüche 1 bis 6, **dadurch gekennzeichnet, dass** die wässrige Formaldehydlösung, von der das Verfahren ausgeht, durch oxidative Dehydrierung von Methanol hergestellt wird.

**Claims**

**1.** A process for preparing high-concentration formaldehyde solutions having a $CH_2O$ content of $\geq$ 50% by weight from an aqueous formaldehyde solution having a lower $CH_2O$ content by evaporation of part of this solution (partial evaporation), in which the aqueous formaldehyde solution is heated to an evaporation temperature T at which the gas phase becomes enriched in water relative to the liquid phase and the gas phase formed is taken off continuously or discontinuously, wherein the evaporation temperature T obeys the relationship:

$$T \; [°C] \; < T_{max} \; [°C]$$

where

$$T_{max}(c) = A + B \; x \; (c/100) + C \; x \; (c/100)^2 + D \; x \; (c/100)^3$$

and
A=+68,759, B=+124,77, C=-12,851, D=-10,095,
and a temperature which obeys the relationship

$$T \; [°C] > T_{min} \; [°C]$$

where

6

$$T_{min}(c) = A' + B' \times (c/100) + C' \times (c/100)^2 + D' \times (c/100)^3$$

and

where A'=+6,0156, B'=+52,918, C'=+49,699, D'=+34,286, in the aqueous formaldehyde solution at every point in the evaporator,

where c is the instantaneous $CH_2O$ content of the aqueous formaldehyde solution during the evaporation in percent by weight and is from 20 to 99% by weight.

2. The process according to claim 1, wherein the aqueous formaldehyde solution used as starting material in the process has a $CH_2O$ content of from 10 to 95% by weight.

3. The process according to claim 2, wherein the aqueous formaldehyde solution used as starting material in the process has a $CH_2O$ content of from 30 to 85% by weight.

4. The process according to any of claims 1 to 3, wherein the pressure during the partial evaporation is from 0.1 to 50 bar.

5. The process according to any of claims 1 to 4, wherein the high-concentration formaldehyde solution obtained has a $CH_2O$ content of from 50 to 99% by weight.

6. The process according to any of claims 1 to 5, wherein the evaporation of the aqueous formaldehyde solution is carried out in a stirred vessel, a helically wound tube, a film evaporator or another apparatus having heat exchanger characteristics.

7. The process according to any of claims 1 to 6, wherein the aqueous formaldehyde solution used as starting material for the process is prepared by oxidative dehydrogenation of methanol.

**Revendications**

1. Procédé pour la fabrication de solutions de formaldéhyde fortement concentrées, ayant une teneur en $CH_2O \geq 50$ % en poids, à partir d'une solution aqueuse de formaldéhyde ayant une teneur en $CH_2O$ plus faible, par évaporation d'une partie de cette solution (évaporation partielle), dans lequel on chauffe la solution aqueuse de formaldéhyde à une température d'évaporation T à laquelle on obtient un enrichissement en eau de la phase gazeuse par rapport à la phase liquide, et on extrait de manière continue ou discontinue la phase gazeuse formée, la température d'évaporation T répondant à la relation suivante :

$$T \, [°C] < T_{max} \, [°C]$$

avec

$$T_{max}(c) = A + B \times (c/100) + C \times (c/100)^2 + D \times (c/100)^3$$

et

A=+68,759, B=+124,77, C=-12,851, D=-10,095,

la solution aqueuse de formaldéhyde étant maintenue, quel que soit l'endroit dans l'évaporateur, à une température constante répondant à la relation suivante :

$$T \, [°C] > T_{min} \, [°C]$$

avec

$$T_{min}(c) = A' + B' \times (c/100) + C' \times (c/100)^2 + D' \times (c/100)^3$$

et

A'=+6,0156, B'=+52,918, C'=+49,699, D'=+34,286,

la valeur c représentant la teneur en $CH_2O$ présente dans la solution aqueuse de formaldéhyde au cours de l'évaporation, exprimée en % en poids, et dont la valeur est de 20 à 99 % en poids.

2. Procédé selon la revendication 1, **caractérisé en ce que** la solution aqueuse de formaldéhyde, avec laquelle on démarre le procédé, présente une teneur en $CH_2O$ de 10 à 95 % en poids.

3. Procédé selon la revendication 2, **caractérisé en ce que** la solution aqueuse de formaldéhyde, avec laquelle on démarre le procédé, présente une teneur en $CH_2O$ de 30 à 85 % en poids.

4. Procédé selon l'une quelconque des revendications 1 à 3, **caractérisé en ce que** la pression exercée au cours de l'évaporation partielle est de 0,1 à 50 bars.

5. Procédé selon l'une quelconque des revendications 1 à 4, **caractérisé en ce que** la solution de formaldéhyde fortement concentrée obtenue présente une teneur en $CH_2O$ de 50 à 99 % en poids.

6. Procédé selon l'une quelconque des revendications 1 à 5, **caractérisé en ce que** l'évaporation de la solution aqueuse de formaldéhyde se fait dans une cuve agitée, un tube spiralé, un évaporateur à film ou un autre appareil doté d'une caractéristique d'échangeur de chaleur.

7. Procédé selon l'une quelconque des revendications 1 à 6, **caractérisé en ce que** la solution aqueuse de formaldéhyde, avec laquelle on démarre le procédé, est obtenue par déshydrogénation oxydative du méthanol.

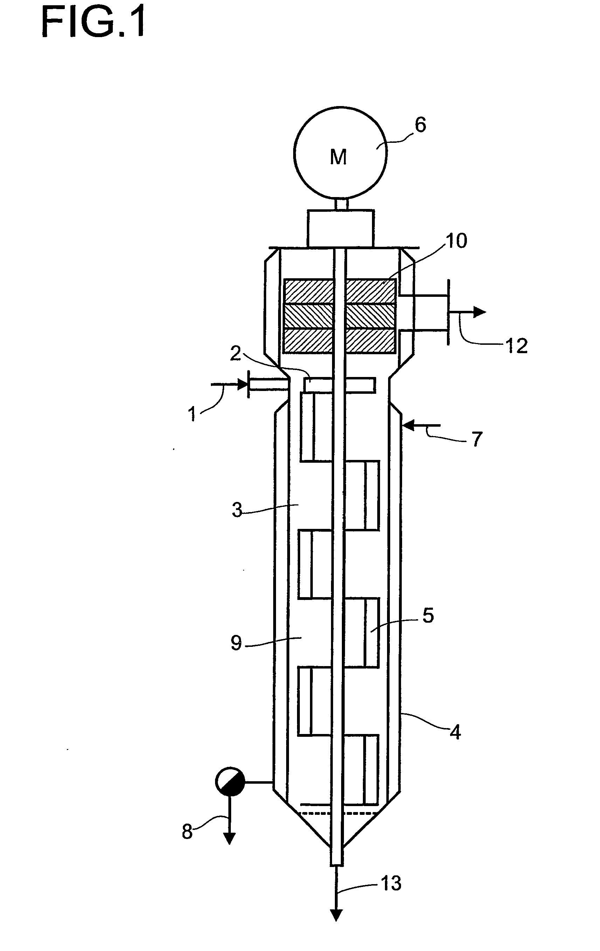

# FIG.1

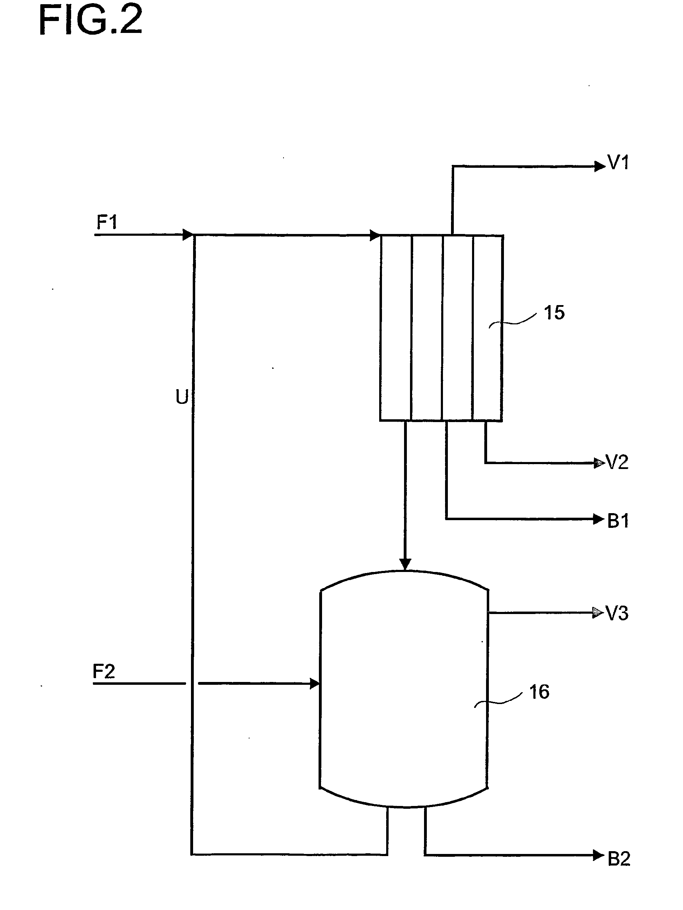

# FIG.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 1190682 A
- EP 1063221 A
- DE 2719967 A
- DE 10158813